# EUROPEAN PATENT APPLICATION

(11) **EP 2 946 759 A1**
(43) Date of publication of application: **25.11.2015**
(21) Application number: 15001433.0
(22) Date of filing: 13.05.2015
(51) Int. Cl.: A61G 13/10, A61G 12/00

(54) **MEDICAL EQUIPMENT SUPPORT SYSTEM FITTED BETWEEN FLOOR AND CEILING**

(30) Priority: 19.05.2014 DE 102014007279
(71) Applicant: The University of Dundee, Dundee, Scotland DD1 4HN (GB)
(72) Inventor: Coleman, Stuart, Dundee Medipark, Dundee DD2 1FD, Scotland (GB); Cuschieri, Alfred, Dundee Medipark, Dundee DD2 1FD, Scotland (GB); Brown, Stuart, Dundee Medipark, Dundee DD2 1FD, Scotland (GB)
(74) Representative: Fugmann, Winfried

(57) **Abstract**

A medical equipment support system comprises a support structure for holding at least one medical device (11), the support structure being configured for being held in a substantially vertical direction by contacting a floor (3) and a ceiling (4) of a medical room, wherein the support structure comprises a vertical column (5) to which at least one holder (6) comprising a substantially horizontal arm is attached for supporting the at least one medical device (11), the substantially horizontal arm being an articulating arm.

## Description

The present invention relates to a medical equipment support system.

The execution of medical care in primary and secondary care settings increasingly relies upon medical devices and technological systems to meet the patient's needs. Such devices are invariably electronic or electromechanical, must be connected to a source of power and possibly to fluids and will occupy space around the patient. In order to avoid congestion of the patient environment with equipment, cables, hoses etc. and in order to improve access by the care staff to the patient, it is well-known to accommodate medical equipment on ceiling-mounted structures. In this way equipment, cables and other connections can be suspended above the workspace where they cause less inconvenience and allow the care-giver unimpeded access to the patient.

In DE 10 2007 051 038 A1 an equipment carrier segment is disclosed that comprises a substantially horizontal supply beam for accommodating a medical device and a base-holder to which the supply beam is mounted. The base-holder is premounted on the ceiling of an operating or intensive care room in a hospital by means of several vertical struts, which are fixed to the ceiling. The base-holder is mounted to the building structure, the struts being adjustable in length in order to compensate for variations in the ceiling height. An equipment support system comprises at least two equipment carrier segments.

According to US 6,089,518 a supportive structure is attached to a ceiling of a hospital room for supporting hospital equipment. The supporting structure comprises beams attached to the ceiling and forming a rectangular space. Inside the space there are non-interchangeable gas connectors and an electric box connected to the supply of the hospital. In EP 0 215 212 A2 a supply unit for a medical nursing post is disclosed that comprises a supply beam fixed to the lower ends of columns hanging from the ceiling of a room. The supply beam comprises a longitudinal rail along which a carriage can be moved from which a carrier device for medical devices is suspended. Supply cables and hoses pass from the ceiling through the hollow columns.

The aforementioned mounting, suspension or support systems rely upon structural features of the ceiling of a medical room where the system is installed. Special facilities and a load bearing structure may be most conveniently incorporated into a building from the outset. If such a system is to be installed retrospectively in an existing building, considerable expenses are incurred for the necessary structural changes to the medical room. Moreover, these are permanent changes, which may not be desirable or practical for all rooms. Such a fixed layout reduces the ability to adapt to changing patient care requirements or changing technology. Since technology for patient care is rapidly evolving, adaptability of accommodation to suit new technologies (i.e. "future proofing") is an increasing need. Moreover, with the continued evolution of medical technology hospitals may need to change the configuration and purpose of rooms several times during the lifetime of a primary hospital building.

It is also known to accommodate medical equipment on a stand structure. According to EP 0 293 227 A2, medical optical equipment is supported by a parallel linkage supported pivotally on a swivel stand shaft set upright for swivel motion on a base. However, such a stand structure often lacks sufficient stability, in particular if heavy-weight medical equipment is to be supported.

In DE 298 11 110 U1 a device for fixation of medical equipment is disclosed, in particular of surgical microscopes in treatment rooms, consisting of a long tube which can be compressed between the floor and the ceiling. For fixation of medical equipment a swivel arm is mounted to the tube. No details relating to the arm are disclosed.

According to DE 20 2006 001 482 U1 a supply column comprises a supply beam extending in a substantially vertical direction. The supply beam has a stand face at its lower end and a fixation device at its upper end, the fixation device being configured for compressing against the ceiling.

In US 4,042,287 a service unit for a doctor's or a dentist's office is described comprising a work bench and one or more cupboards, rotatably supported on a pillar stand which is mainly of U shape. The pillar stand is provided with a pillar base and a top support which are both designed as clamping means for enabling the stand to be vertically arrested between the floor and the ceiling of a room.

It is an object of the present invention to provide a medical equipment support system which alleviates the above mentioned problems. In particular, it is an object of the invention to provide a medical equipment support system that is not or is less dependent on dedicated permanent structures of a medical room in which it is placed, for example in an operating theatre, and which provides sufficient stability, can be handled easily and can be adapted to a variety of needs in a medical room.

This object is met by a medical equipment support system according to claim 1. Advantageous embodiments of the present invention are indicated in the dependent claims.

According to the present invention, a medical equipment support system comprises a support structure which is configured for holding at least one medical device. The medical device can be held directly or indirectly by the support structure, and may be in a fixed or in a variable relationship to the support structure. According to the invention, the support structure is configured for being held by mechanically contacting a floor and a ceiling of a medical room, such that it extends substantially in a vertical direction between the floor and the ceiling. Preferably, any deviation from an exactly vertical direction is zero or small enough such that stability is not reduced. The support structure thus forms a vertical pillar between the floor and the ceiling, transferring a load of the support structure and the at least one medical device to the floor and being laterally stabilized by the ceiling.

The support structure further comprises an elongated column configured to be arranged in the substantially vertical direction. To this vertical column at least one holder is attached for supporting the at least one medical device. Preferably, the support structure is a vertical column to which the at least one holder is attached. The holder may be permanently attached to the column or may be attachable to and removable from the column. Preferably, the holder is configured for being fixed to the column at a multiplicity of vertical positions among which a position can be chosen which fits best to the needs of a particular medical intervention. The column may be configured for being equipped with a multiplicity of holders. Further, the at least one holder comprises a substantially horizontal arm to which the medical device can be mounted. The substantially horizontal arm is an articulated arm which may comprise one or more hinges for adjusting a position and/or orientation of the at least one medical device mounted to the arm.

Due to the support structure not only standing on the floor of the medical room, but also contacting the ceiling, sufficient lateral stability can be achieved for supporting medical equipment which may have considerable weight and may need to be held at various positions. The medical equipment support system can easily be retro-fitted into any room of a building by establishing mechanical contact to the floor and to the ceiling of the medical room, without requiring expensive reconstruction of the room. In particular, no retro-fitting of mechanical support structures into the ceiling of the room is necessary. Floors of medical rooms frequently have sufficient load capacity for carrying the support system. The system can be installed in a medical room without any, or with only a minimum of permanent changes to the existing fabric of the building. In this way it is easy to re-configure a room for a different purpose. For example, with a minimum of changes a room which was once a patient examination room can be changed to an x-ray suite or an anaesthetic bay, etc. The support system can be configured as a non-permanent, adjustable, mobile and/or modular system. The support system provides the benefits of simplicity and minimal bulk, and in particular allows rapid assembly and removal, adaptability for a variety of applications, ease of transport, storage and cleaning and the possibility of varying functions by virtue of optional accessories. Due to the support structure comprising or being configured as a vertical column, the support is simple and can be transported and installed easily. Further, due to at least one holder being attached to the vertical column, the holder comprising a substantially horizontal arm which is an articulated arm, handling of the support system is facilitated, and versatility increased. The support system may be usable in conjunction with pre-installed overhead lighting and equipment, and in conjunction with further support systems.

Preferably, a vertical dimension, i.e. a length of the support structure, in particular the length of the vertical column, can be adapted for fitting tightly between the floor and the ceiling of the medical room. To this end, hydraulic, pneumatic, or mechanical means can be employed, for example a screw drive or a ratchet for adapting the length of the column. Thus, a tight fit between the floor and the ceiling can be achieved to improve stability of the medical equipment support system.

Most preferably, the support structure is configured for being compressed between the floor and the ceiling of the medical room. In this way a friction fit to the floor and to the ceiling can be achieved, such that the support structure is firmly held between the floor and the ceiling. The rigidity of the friction fit can be adjusted by adjusting the compressive force acting upon the support structure. In this way a high degree of stability can be achieved such that the support system is able to accommodate large loads and moments, while no permanent structural changes to the floor or the ceiling are required. The support structure, in particular the vertical column, preferably is dimensioned to avoid buckling even under large compressive forces and lateral moments.

According to a preferred embodiment of the invention the elongated column configured to be arranged substantially in the vertical direction between the floor and the ceiling has an enlarged floor and/or ceiling contact area. More preferably, the support structure is a vertical column with enlarged floor and/or ceiling contact areas. The contact areas may be defined by a foot plate (plinth) or a head plate (capital). Alternatively, the contact area may be defined by a multiplicity of struts which protrude to the lateral of the column close to its lower and/or upper end to support the column against the floor or ceiling, respectively. Moreover, one or more struts may be provided in addition to a foot plate and/or head plate to increase at least one dimension of the floor or ceiling contact area, respectively. Due to the enlarged contact areas, the stability of the support system is further enhanced, and the load may be distributed over a larger area of the floor and/or ceiling. If the support structure is fixed by being compressed between the floor and the ceiling of the room, such distribution of the force is most advantageous in order to limit the load per area.

According to a particularly preferred embodiment, the ceiling contact area is larger than the floor contact area. Thus, for example, the column may be equipped with a foot plate and a head plate, wherein the head plate is larger than the foot plate. Alternatively, for example, the column may have a foot plate at its lower end and a number of struts at its upper end, the struts encompassing a contact area to the ceiling which is larger than the area of the foot plate. Due to the ceiling contact area exceeding the floor contact area, stability of the support system can be maximized, while the degree of impediment imposed to medical staff by a foot plate or struts arranged at the lower end of the column is minimized.

According to a preferred embodiment of the invention, the support structure, and preferably the at least one holder attached to the support structure, is configured for accommodating cables and/or fluid lines. The cables may be electric cables for transmission of electric power and/or signals, as well as optical cables for transmission of illumination light or endoscopic images, for example. The fluid lines may comprise, for example, gas supply for insufflation or respiration or lines for irrigation fluid and suction. In particular, the support structure may comprise a hollow space wherein the cables and the fluid lines can be installed and which may be accessible by removable hatches. The vertical column may be a hollow column, for example, the hollow inside of which allows transit of cables and/or fluid lines, lateral holes in the column being provided for routing the cables and/or fluid lines into or out of the column. Moreover, a foot or head plate may be employed for connections to connectors installed fixedly in the floor or ceiling of the medical room, respectively. Thus, supply of the medical equipment with electricity, signals, fluids, etc. is permitted while medical supply equipment can be placed away from the medical device, avoiding obstruction of available operating space and keeping the supply equipment outside the sterile zone during a surgical procedure.

Further it may be advantageous that the support structure comprises a cabinet which is adapted for placing medical equipment in it. In this way the equipment can be made readily available to the medical staff without interfering more than necessary with the medical procedures. In particular, standard medical supply equipment can be placed within the cabinet, being connected to the medical devices by cables and fluid lines running through the support structure and the holder including the substantially horizontal arm to which at least one medical device is mounted.

According to a preferred embodiment of the invention the articulating arm comprises a substantially horizontal bar carrying a swiveling lever. In particular, the horizontal bar may be rigidly fixed on its first end to a mounting block being attached to the vertical column. Further, the horizontal bar preferably carries a hinge at or close to its second end, which is its distant end as seen from the column. By means of the hinge, a lever is mounted to the bar such that the lever can be swiveled, the axis of the hinge being substantially horizontal and transverse to a longitudinal axis of the bar. The hinge may be located spaced from both ends of the lever, thus forming two sections of the lever, which may be of unequal lengths. Medical equipment may be mounted to one or both sections, in particular to one or both ends of the lever. The medical equipment and/or the lengths of both sections may be chosen to achieve compensation of weights. Thus, in a simple way medical equipment can be held such that it can be easily manipulated by medical staff into a variety of positions and orientations.

Preferably, the medical equipment support system comprises a weight compensation for compensating for the moment tilting and/or bending the column, exerted by a load carried by the substantially horizontal arm. In particular, the holder may be equipped with a counter-weight to compensate for the weight of the arm and a medical device mounted to the arm. In this way buckling of the column can be avoided even when heavy equipment is held on an arm at a considerable horizontal distance from the column. The horizontal distance from the column at which the counter-weight is held may be adjustable.

According to a preferred embodiment, the support structure comprises a rail, and the at least one holder is movable along the rail. In particular, the support structure may be a vertical column forming a vertical rail, along which at least one holder can be moved manually, by a motorized drive, or automatically under computer control. In this way, a medical device mounted to the holder can easily be positioned at a height above the floor which is convenient for operation by medical staff. Moreover, the at least one holder itself offers additional degrees of freedom for adjusting a position and/or orientation of the medical device, the holder being adjustable manually, by a motorized drive, or automatically under computer control, in particular by adjusting the articulating arm. In this way, movement of medical devices in a substantially horizontal direction as well as rotational movement of medical devices is enabled.

Advantageously, the holder is movable into a preference position which is defined within a predetermined medical scheme. In particular, the medical scheme may be a scheme of positions and orientations of medical devices required in a particular medical procedure, for example in a particular surgical procedure. In order to reach the preference position, the holder is movable along the rail and/or the holder itself is adjustable with respect to its own degrees of freedom. By moving the holder into the preference position the medical device carried by the holder is placed in a preference position and orientation as determined by the medical scheme. There may be more than one preference position and orientation for each medical device. In this way moving the medical devices required in a particular medical procedure into positions and orientations required by the medical procedure is facilitated.

According to a preferred embodiment, the holder is manually movable, the at least one preference position being defined by a stop. In particular, the stop may be haptically sensible when the holder is manually moved along the rail. The haptic sensing of the stop can be achieved by an increased resistance or a detent being provided on the rail at the preference position. The position of the stop may be automatically adjustable, in particular the stops may be powered, so that they can be moved automatically into position under computer control. The stop or stops may be designed to allow some holders to pass unhindered and stop only specific, predetermined holders which may be configured for holding a particular kind of medical equipment. In a similar way, a stop may be provided within the holder if the holder is adjustable in itself, in particular one or several stops may be provided within the hinge or hinges of the articulating arm. Preferably, a compensation of the weight of a holder and the medical equipment carried by the holder may be provided to facilitate manual movement along the vertical rail. By means of the manual movement, the position and orientation of the medical devices can be controlled by the medical staff in an optimal manner, while reaching of a predetermined preference position is facilitated.

According to another preferred embodiment of the invention, the holder is automatically movable into the at least one preference position. This may be achieved by means of a drive system and a sensor system. The drive system is configured for moving the holder along the rail and for adjusting the holder itself, in particular for adjusting the articulating arm in one or more degrees of freedom. The sensor system is configured for detecting when the preference position has been reached. A control system may be provided for controlling the drive system and the sensor system such that when the preference position has been reached the drive system is automatically stopped. Preferably, the control system is also configured for controlling the medical devices used in the medical procedure. Due to the holder being automatically movable into the preference position, an optimal arrangement of medical devices can be most easily achieved before and during a medical procedure.

Preferably, the system design is modular so that it can be tailored to the specific requirements of a particular operating theatre. Each module is small enough to be transported through the doors of the theatre, after which the modules may be assembled in situ. The modules may include the column, optionally with a built-in length adjustment mechanism, one or more holders for mounting medical equipment, and one or more equipment cabinets, and stabilising foot or head plates or struts. In particular, the modules are designed to be releasably connectable to each other and to be transportable into an operating theatre through standard access doors. In this way, optimal versatility of the support system can be achieved.

According to a further aspect of the invention, the medical equipment support system is configured according to the generic clause of claim 1, but is not necessarily limited to the features of the characterizing clause of claim 1. Thus, according to this aspect of the invention, a medical equipment support system comprises a support structure for holding at least one medical device, the support structure being configured for being held in a substantially vertical direction by contacting a floor and a ceiling of a medical room. According to this aspect of the invention, the support structure may be configured such that it does not comprise a vertical column to which at least one holder comprising a substantially horizontal arm is attached for supporting the at least one medical device and/or the substantially horizontal arm may be configured in another way than as an articulating arm. With respect to other features the medical equipment support system preferably is configured as described above, in particular it may be configured as claimed in the dependent claims.

The features of the invention as mentioned above and as described below apply not only in the combinations mentioned but also in other combinations or alone, without leaving the scope of the present invention.

Further aspects of the present invention will be apparent from the figures and from the description of particular embodiments that follows.
Fig. 1 shows a first embodiment of the invention in a side view in an operating theatre;
Fig. 2 is a perspective view of the embodiment of Fig. 1 with the ceiling of the operating theatre removed;
Fig. 3 illustrates a length adjustment mechanism incorporated in the embodiment of Figs. 1 and 2;
Fig. 4 illustrates another length adjustment mechanism;
Fig. 5 shows a second embodiment of the invention in a side view in an operating theatre.

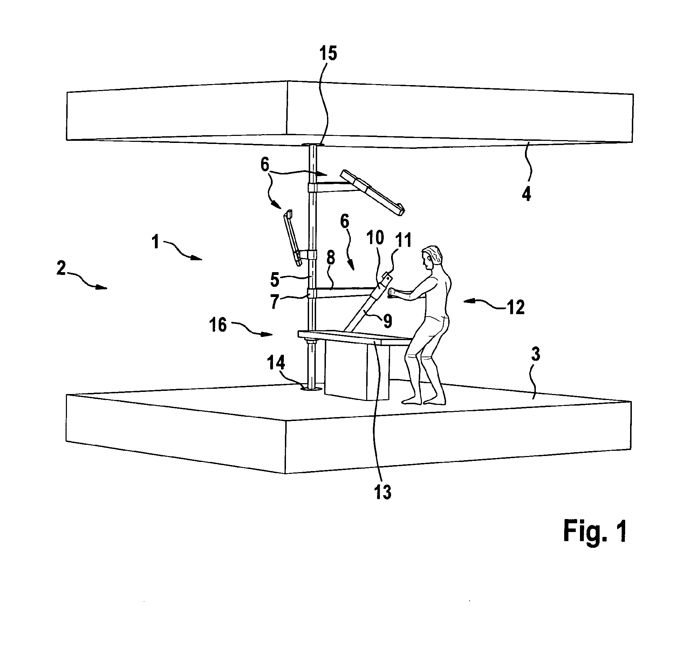

As shown in Fig. 1, a medical equipment support system 1 according to a first embodiment of the invention is set up in a medical room, which may be an operating theatre 2. The walls of the operating theatre 2 are omitted in Fig. 1. The support system 1 forms a single pillar standing at a distance to the walls of the operating theatre 2 and locked in a vertical position between the floor 3 and the ceiling 4. The support system 1 comprises a vertical column 5 to which a number of holders 6 are attached. Each holder 6 comprises a mounting block 7 being movably attached to the column 5, holding a bar 8 in a horizontal direction. At its distant end the bar 8 is provided with a swiveling lever 9 connected to the bar 8 by means of a hinge 10. The hinge 10 divides the lever 9 unequally to define a short end and a long end of the lever 9 to one or both of which a medical device 11 can be mounted. Preferably, the position of the hinge 10 on the lever 9 can be adjusted. The medical device 11 may be mounted to the lever 9 via one or more further hinges. If a medical device is mounted on the short end and another medical device is mounted on the long end of the lever 9, the position of the hinge 10 on the lever may be chosen such that both medical devices compensate each other's weight; alternatively, instead of one of the medical devices a dedicated counter-mass may be mounted on the opposing end of the lever 9.

The bar 8 and the lever 9 form a substantially horizontal arm, which is an articulating arm, for holding the medical device 11 at a variety of positions and orientations. The height of the horizontal bar 8 can be chosen by moving the mounting block 7 along the column 5. A multiplicity of holders 6 may be attached to the column 5 at the same time in order to increase the number of medical devices supported. Positions and orientations of the medical devices 11 mounted to the holders 6 can thus be adjusted for convenient use by medical staff 12 in a surgical procedure conducted on a patient placed on an operating table 13 situated adjacent to the column 5. The mounting block 7 may be fixed at a desired position on the column 5, as well as the hinge 10 and possibly the one or more further hinges may be fixed in a desired angular orientation. It may be advantageous to include a fail-safe feature such as a bolt to lock the mounting block in place when in use, so that it may not under any circumstances move undesirably. The mounting block 7 may be configured for allowing turning the bar 8 around the column 5 in order to remove a medical device 11 from the vicinity of the operating table 13 if it is currently not needed. Conversely, the holder 6 can be turned towards the operating table 13 if required.

In a simple example, the column 5 may be a cylindrical steel tube with a length that corresponds to the height of the ceiling of a room where the support system is to be installed, with an outer diameter and wall thickness sufficiently large to prevent column buckling. The hollow inside of the column 5 allows for transit of fluids, cabling etc. In this case the mounting blocks 7 of the holders 6 would be cylindrical sleeves movable along and rotatable around the column 5, with a locking mechanism for fixation on the column 5 in a desired position and orientation. The bars 8 may be fixedly or removably connected to the respective mounting block 7.

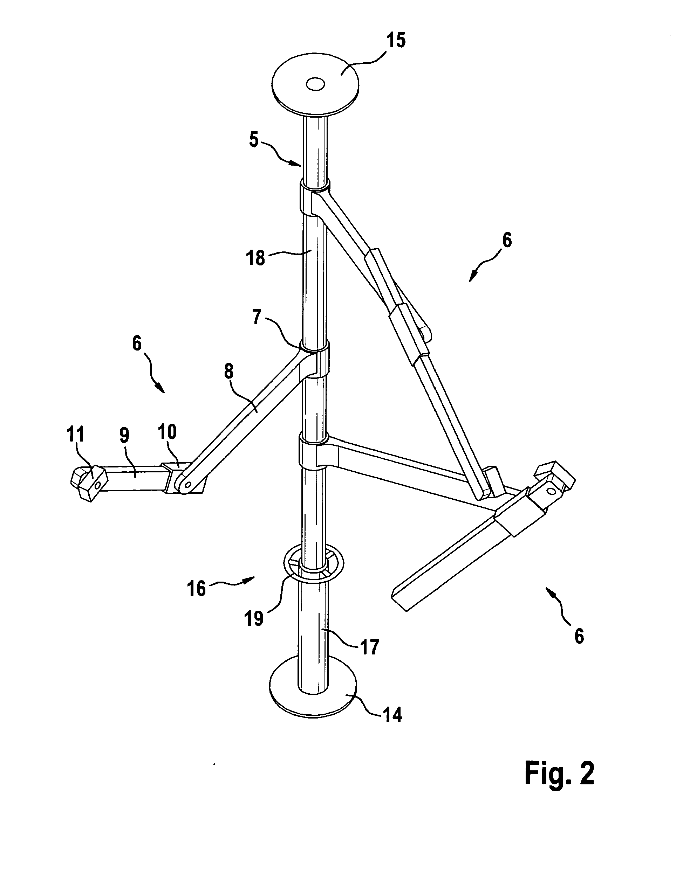

In order to improve fixation of the column 5 between the floor 3 and the ceiling 4 of the operating theatre 2, the column 5 is equipped with a foot plate 14 and a head plate 15, as well as with a length adjustment mechanism 16, which are explained in more detail referring to Fig. 2.

In the embodiment illustrated in Figs. 1 and 2, the foot plate 14 and the head plate 15 are circular plates each having a diameter which is enlarged with respect to the diameter of the column 5. Thus, a force exerted by the support system 1 upon floor 3 and ceiling 4 (see Fig. 1) due to its weight, including medical equipment, and due to compression, is distributed upon a larger area. The foot plate 14 on its lower side and the head plate 15 on its upper side may be equipped with a surface structure or material which is chosen to increase friction with the floor and the ceiling, respectively. In this way, stability of the support system 1 may be improved as well as its capability of supporting medical equipment which may have considerable weight and may be held, depending on dimensions and adjustment of the bar 8 and the lever 9, at a considerable horizontal distance from the column 5. In order to adjust the length of the column 5 to the height of the operating theatre 2 and, by further adjustment, to build up sufficient compressive force, the column 5 is divided into a lower section 17 and an upper section 18 one of which is guided upon the other to make the column 5 length-adjustable. The length adjustment mechanism 16 may be operated by a hand wheel 19.

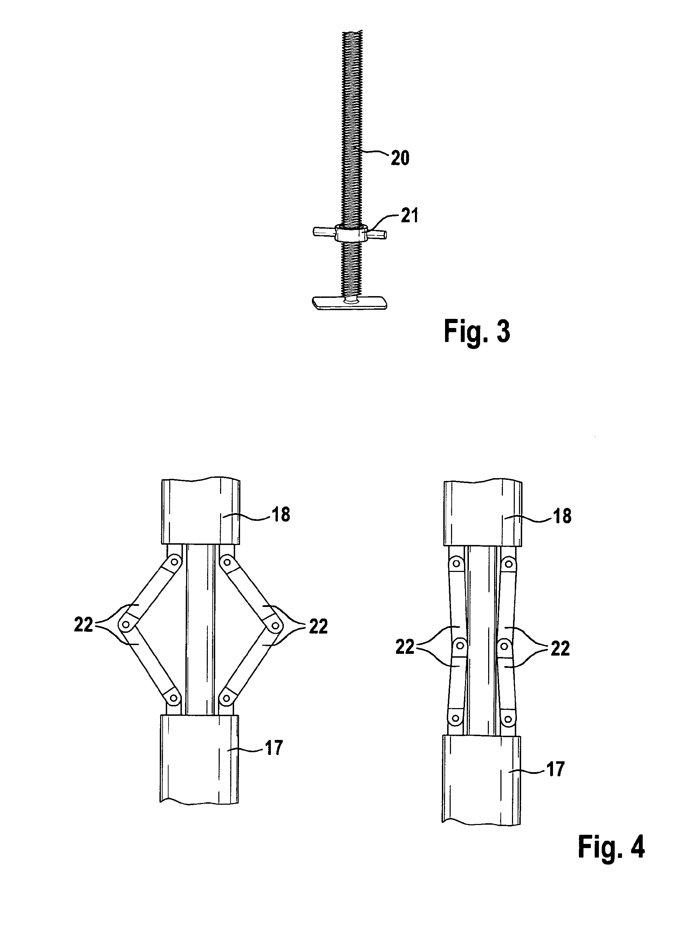

As shown symbolically in Fig. 3, the length adjustment mechanism may be configured like a screw-jack, comprising a threaded rod 20 formed integrally with the lower section 17 of the column 5 and a nut 21 upon which the upper section 18 rests. The nut 21 can be turned by the hand wheel 19 in order to lower or raise the upper section 18 of the column 5 with respect to the lower section 17 (see Figs. 1 and 2). In this way, the length of the column 5 can be adjusted to fit the height of the operating theatre 2, and by further turning of the hand wheel to build up compressive force for creating a firm friction fit of the support system 1 between floor 3 and ceiling 4.

Additionally or alternatively, an over-centre mechanism with a multiplicity of hinged levers 22 can be employed for quick adjustment of the length of the column 5 and for building up compressive force. Fig. 4 shows an exemplary embodiment of such an over-centre mechanism in its pre-mounting state (left) and its mounted state (right).

Preferably, the column 5 is capable of being easily extended until it is in compression. A means of achieving this is to provide a coarse adjustment with a screw jack mechanism as shown in Fig. 3 and then use an over-centre mechanism as depicted in Fig. 4 to create the final compressive force. Conversely, if the column 5 is provided in a length which roughly corresponds to the height of an operating theatre 2, an over-centre mechanism as depicted in Fig. 4 may be employed for preliminary fixation and a jack mechanism such as shown in Fig. 3 for build-up of the final compressive force. Moreover, a compressive spring may be arranged between the lower and upper sections 17, 18 of the column 5 in order to provide a compressive force for preliminary fixation during assembly of the support system. Alternatively or additionally, a compressive spring may be arranged in series to the length adjustment mechanism 16 to limit the total achievable compressive force.

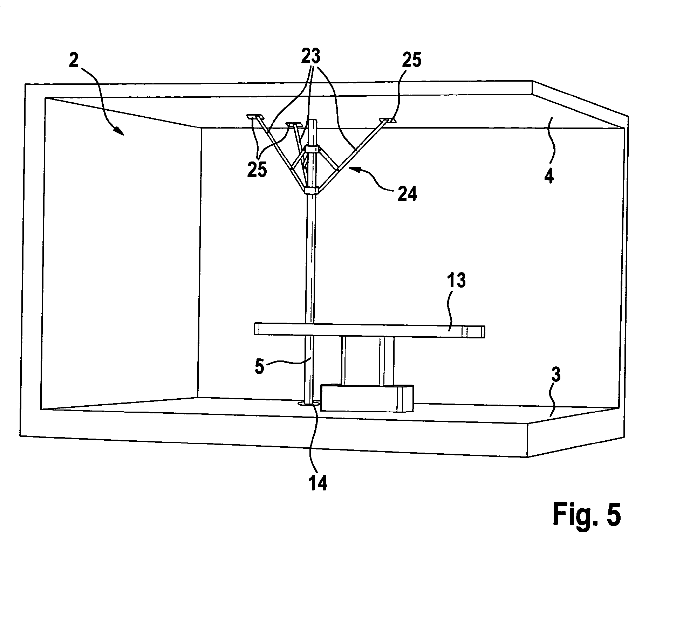

According to a second embodiment of the invention and as illustrated in Fig. 5, the head plate is replaced by a multiplicity of struts 23. In particular, the struts 23 may form a tripod defining a triangular area on the ceiling 4. The struts 23 may be collapsed for easy transport and spread by a spreading mechanism 24. Each strut may comprise an adjustable end plate 25 for distributing load. The end plates 25 may be provided with a surface structure or material for improving the friction fit to the ceiling 4. The struts 23 with their end plates 25 are pressed against the ceiling 4 by a length adjustment mechanism (not shown). The length mechanism, which also serves to build up the compressive force for firm fixation between floor 3 and ceiling 4, may be arranged at the junction of the struts 23 to the column 5, or may be configured as described above. In relation to details not shown in Fig. 5 reference is made to Figs. 1 and 2. By replacing or, possibly, supplementing the head plate by the struts 23, the effective area of the ceiling 4 employed for fixation of the column 5 is enlarged. Stability and load carrying capacity of the support system can thus be increased without increasing the area occupied by the foot plate 14.

In any one of the embodiments described, the design may be modular so that it can be tailored to the specific requirements and dimensions of a particular operating theatre. All modules of the system are sufficiently small that they may be transported into an operating theatre through the standard access doors, and the components can be assembled relatively quickly in situ. The modules may be the column 5 including a length adjustment mechanism 16, the holders 6, the head and foot plates 14, 15 and/or an arrangement of struts 23. The column 5 may be available in a range of lengths so that it could be easily tailored to the height of the operating theatre; if necessary the column 5 can be assembled from multiple parts, for example from lower and upper sections 17, 18 in situ. The support system 1 remains in place as long as required but may be readily removed or adjusted to permit the use of a given room or space to be altered without permanent structural or architectural alterations to the building. All connections preferably are non-permanent so that the assembled structure can be disassembled for transport or reconfiguration at a later date.

In any one of the embodiments described, medical equipment carried by the medical equipment support system 1 on the holders 6 may be, for example, lighting, power sockets or socket posts which provide multiple cable plug-ins, connections to vacuum, insufflation and medical gasses, irrigation, fluid drainage, electrosurgery devices and anaesthetics, instrument holders, floating table, instrument tray, visual display unit (monitor), raised storages shelves, patient hoist, armrest and/or seat, isolation transformer, operating microscope, patient monitoring equipment such as pulse oximeter, electrocardiography, etc. and/or imaging equipment such as for fluoroscopy. Moreover, the medical equipment support system 1 may be used in conjunction with overhead lighting and equipment and overhead attachments for drapes, covers or curtains installed on the ceiling 4 of the operating theatre 2.

While the embodiments of the invention have been described in relation to use in an operating theatre, the medical equipment support system may be employed as well in other care-giving settings such as treatment rooms, hospital wards, imaging facilities, storage areas, etc.

For clarity not all reference numerals are displayed in all figures. If a reference numeral is not explicitly mentioned in the description of a figure, it has the same meaning as in the other figures.

### Reference numerals

- 1: Support system
- 2: Operating theatre
- 3: Floor
- 4: Ceiling
- 5: Column
- 6: Holder
- 7: Mounting block
- 8: Bar
- 9: Lever
- 10: Hinge
- 11: Medical device
- 12: Medical staff
- 13: Operating table
- 14: Foot plate
- 15: Head plate
- 16: Length adjustment mechanism
- 17: Lower section
- 18: Upper section
- 19: Hand wheel
- 20: Threaded rod
- 21: Nut
- 22: Lever
- 23: Strut
- 24: Spreading mechanism
- 25: End plate

## Claims

1. Medical equipment support system, comprising a support structure for holding at least one medical device (11), the support structure being configured for being held in a substantially vertical direction by contacting a floor (3) and a ceiling (4) of a medical room, **characterized in that** the support structure comprises a vertical column (5) to which at least one holder (6) comprising a substantially horizontal arm is attached for supporting the at least one medical device (11), the substantially horizontal arm being an articulating arm.

2. Medical equipment support system according claim 1, **characterized in that** a length of the support structure can be adapted for fitting tightly between the floor (3) and the ceiling (4) of the medical room.

3. Medical equipment support system according to any one of the preceding claims, **characterized in that** the support structure is configured to be compressed between the floor (3) and the ceiling (4) of the medical room.

4. Medical equipment support system according to any one of the preceding claims, **characterized in that** the vertical column (5) has an enlarged floor and/or ceiling contact area.

5. Medical equipment support system according to claim 4, **characterized in that** the vertical column (5) is equipped with a foot plate (14) and/or a head plate (15) to contact the floor (3) and/or ceiling (4), respectively.

6. Medical equipment support system according to claim 4 or 5, **characterized in that** the vertical column (5) is equipped with a multiplicity of laterally spreading struts (23) at its lower and/or upper end to contact the floor (3) and/or ceiling (4), respectively.

7. Medical equipment support system according to one of claims 4-6, **characterized in that** the ceiling contact area is larger than the floor contact area.

8. Medical equipment support system according to any one of the preceding claims, **characterized in that** the support structure is configured for accommodating cables and/or fluid lines.

9. Medical equipment support system according to any one of the preceding claims, **characterized in that** the support structure comprises a cabinet for placing medical equipment.

10. Medical equipment support system according to any one of the preceding claims, **characterized in that** the articulating arm comprises a substantially horizontal bar (8) carrying a swiveling lever (9).

11. Medical equipment support system according to any one of the preceding claims, **characterized in that** the support system comprises a weight compensation for compensating a load carried by the substantially horizontal arm.

12. Medical equipment support system according to any one of the preceding claims, **characterized in that** the support structure comprises a rail, and that the at least one holder (6) is movable along the rail.

13. Medical equipment support system according to claim 12, **characterized in that** the holder (6) is movable into a preference position according to a predetermined medical scheme.

14. Medical equipment support system according to claim 13, **characterized in that** the holder (6) is manually movable into the preference position, the preference position being indicated by a stop.

15. Medical equipment support system according to claim 13 or 14, **characterized in that** the holder (6) is automatically movable into the preference position.
